# EUROPEAN PATENT SPECIFICATION

(11) **EP 3 119 750 B1**
(45) Date of publication and mention of the grant of the patent: **08.05.2019**
(21) Application number: 15723054.1
(22) Date of filing: 18.03.2015
(51) Int. Cl.: C07D 307/42, C08K 5/1535

(54) **PENDANT FURYL CONTAINING BISPHENOLS, POLYMERS THEREFROM AND A PROCESS FOR THE PREPARATION THEREOF**
HÄNGENDE FURYLHALTIGE BISPHENOLE, POLYMERE DARAUS UND VERFAHREN ZUR HERSTELLUNG DAVON
BISPHÉNOLS CONTENANT UN GROUPE FURYLE PENDANT, POLYMÈRES PROVENANT DE CEUX-CI ET PROCÉDÉ DE PRÉPARATION CORRESPONDANT

(30) Priority: 18.03.2014 IN 791DE2014
(43) Date of publication of application: 25.01.2017
(73) Proprietor: Council of Scientific and Industrial Research, New Delhi, Maharashtra 110001 (IN)
(72) Inventor: KUHIRE, Sachin Suresh, Pune Maharashtra 411 008 (IN); NAGANE, Samadhan Suresh, Pune Maharashtra 411 008 (IN); WADGAONKAR, Prakash Purushottam, Pune Maharashtra 411 008 (IN)
(74) Representative: J A Kemp
(86) International application number: PCT/IN2015/000132
(87) International publication number: WO 2015/140818

(56) References cited:
- WO-A1-2014/073001
- WANG K J ET AL: "New norlignan derivatives from Curculigo capitulata", FITOTERAPIA, IDB HOLDING, MILAN, IT, vol. 81, no. 7, 1 October 2010 (2010-10-01), pages 869-872, XP027275838, ISSN: 0367-326X [retrieved on 2010-05-23]
- BABU B MADHU ET AL: "A rapid, highly efficient, and general protocol for the synthesis of functionalized triarylmethanes: a straightforward access for the synthesis of (-)-tatarino", TETRAHEDRON LETTERS, vol. 55, no. 11, 31 January 2014 (2014-01-31), pages 1868-1872, XP028624820, ISSN: 0040-4039, DOI: 10.1016/J.TETLET.2014.01.107
- STEFANIE KREPPENHOFER ET AL: "Identification of (furan-2-yl)methylated benzene diols and triols as a novel class of bitter compounds in roasted coffee", FOOD CHEMISTRY, ELSEVIER LTD, NL, vol. 126, no. 2, 1 November 2010 (2010-11-01), pages 441-449, XP028210986, ISSN: 0308-8146, DOI: 10.1016/J.FOODCHEM.2010.11.008 [retrieved on 2010-11-05]
- PANDURANG N. HONKHAMBE ET AL: "Synthesis and characterization of new aromatic polyesters containing pendent naphthyl units", JOURNAL OF APPLIED POLYMER SCIENCE, 1 January 2010 (2010-01-01), pages NA-NA, XP055201464, ISSN: 0021-8995, DOI: 10.1002/app.32162
- WANG AILING ET AL: "Brönsted acid ionic liquids catalyzed Friedel-Crafts Alkylations of electron-rich arenes with aldehy", APPLIED CATALYSIS A: GENERAL, ELSEVIER SCIENCE, AMSTERDAM, NL, vol. 482, 2 June 2014 (2014-06-02), pages 198-204, XP029040365, ISSN: 0926-860X, DOI: 10.1016/J.APCATA.2014.05.014

## Description

### FIELD OF THE INVENTION:

The present invention relates to a pendant furyl containing bisphenols formula (I), polymers therefrom and a process for the preparation thereof. More particularly, the present invention relates to a novel bisphenols of formula (I) which are useful for preparation of polymers with pendant furyl groups and a process for synthesis of the bisphenols and polymers therefrom.

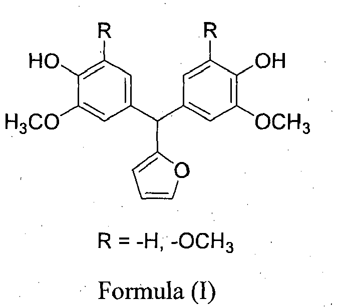

### BACKGROUND AND PRIOR ART OF THE INVENTION:

Lignocellulose which comprises of lignin, cellulose and hemicellulose is an abundantly available and non-edible biomass resource useful for the production of value-added chemicals. Over 70 million tons of lignin is produced annually in the world as a byproduct of paper mills and 95 % of that production is burnt to produce heat and/or electricity within paper mills and biorefinery. Lignin extracted from wood and agricultural residues like wheat straw, sugar cane bagasse, etc upon depolymerization yields aromatics such as vanillin, syringaldehyde, syringol, guaiacol, vanillic acid, syringic acid, ferulic acid, etc. Another lignocellulose fraction contains cellulose and hemicellulose which are converted into useful furan derivatives such as furfural, 5-hydroxylmethyl furfural and so on.

Bisphenols are an important class of difunctional monomers which find applications in the preparation of a host of high performance polymers such as polycarbonates, polyesters, polyether sulfones, polyether ether ketones, epoxy resins, etc. Current estimates indicate that more than 4 million tons of Bisphenol-A is produced annually from petroleum-based chemicals. However, Bisphenol-A has come under scrutiny due to its potential health hazards. Therefore, the global polymer community's research efforts are focused on replacement for Bisphenol-A.

Most of the reported bisphenols are synthesized from starting materials that are petrochemical-based. Some partially bio-derived bisphenols are known in the prior art wherein starting materials such as levulinic acid, limonene, cashew nut shell liquid, creosol, 2,6-dimethoxy phenol, etc have been made use of.

Article titled "Synthesis and characterization of new aromatic polyesters containing pendant naphthyl units" by Honkhambe et al. published in Journal of Applied Polymer Science, 2010, 117 (5), pp 2545-2552 reports two bisphenols, viz., 4,4'-[1-(2-naphthalenyl)ethylidene]bisphenol and 4,4'-[1-(2-naphthalenyl) ethylidene]bis-3-methylphenol which are prepared by condensation of commercially available 2-acetonaphthanone with phenol and o-cresol, respectively. It reports a series of new aromatic polyesters containing pendant naphthyl units synthesized by phase-transfer-catalyzed interfacial polycondensation of these bisphenols with isophthaloyl chloride, terephthaloyl chloride, and a mixture of isophthaloyl chloride : terephthaloyl chloride (50 : 50 mol %).

Article titled "Synthesis and characterization of new aromatic polyesters containing cardo decahydronaphthalene groups" by Honkhambe et al. published in European Polymer Journal, 2010, 46, pp 709-718 reports synthesis of three cardo bisphenols containing decahydronaphthalene group starting from commercially available 2-naphthol. These bisphenols were utilized for synthesis of new aromatic polyesters by phase transfer-catalyzed interfacial polycondensation with isophthaloyl chloride, terephthaloyl chloride and a mixture of isophthaloyl chloride and terephthaloyl chloride (50:50 mol %).

Article titled "Renewable alternating aliphatic-aromatic copolyesters derived from biobased ferulic acid, diols, and diacids: sustainable polymers with tunable thermal properties" by Pion et al. published in Macromolecular Chemistry and Physics, 2014, 215 (5), pages 431-439 reports alternating aliphatic-aromatic copolyesters which are obtained through the polycondensation of bio-based diacyl chlorides and bisphenols derived from ferulic acid and biosourced diols. Four linear polymeric structures were obtained by polycondensation of two bio-based bisphenols and two diacyl chlorides (succinyl or azelaoyl chloride). These polycondensations were conducted at 100-180°C under inert atmosphere and studied in bulk as well as in solvent.

Article titled "Synthesis of renewable bisphenols from creosol" by Meylemans et al. published in ChemSusChem, 2012, 5(1), pp 206-10 reports a series of renewable bisphenols synthesized from creosol (2-methoxy-4-methylphenol) through stoichiometric condensation with short-chain aldehydes. Creosol can be readily produced from lignin, potentially allowing for the large scale synthesis of bisphenol A replacements from abundant waste biomass. Zinc acetate was shown to be a selective catalyst for the ortho-coupling of formaldehyde, but was unreactive when more sterically demanding aldehydes were used.

Article titled "Synthesis of aromatic polyesters with pendant carboxyl groups from diphenolic acid, bisphenol A and isophthaloyl chloride by interfacial polycondensation" by Zhang et al. published in Advanced Materials Research, 2011, 239-242, pp 2616-2619 reports aromatic polyesters bearing pendant carboxyl functionalities prepared by interfacial polycondensation of diphenolic acid, bisphenol A and isophthaloyl chloride with tetrabutylammonium chloride as a phase transfer catalyst.

However, there is no prior art available that utilizes raw materials from completely bio-based resources for synthesis of bisphenols containing clickable group namely pendant furyl group.

### OBJECTIVE OF INVENTION:

The main objective of present invention is to provide pendant furyl containing bisphenols of formula (I), polymers therefrom and a process for the preparation thereof.

Another objective of present invention is to provide a process for synthesis of bisphenols of formula (I) containing pendant 'clickable' furyl group and self-healable high performance polymers therefrom.

### SUMMARY OF THE INVENTION:

Accordingly, the present invention provides bisphenol compounds of formula (I) 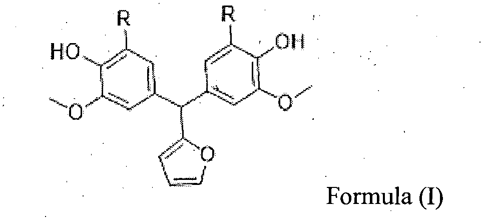 Wherein R is hydrogen or methoxy.

In an embodiment of the present invention the compounds are selected from 4, 4'-(furan-2-ylmethylene) bis(2,6-dimethoxyphenol) and 4,4'-(furan-2-ylmethylene)bis(2-methoxyphenol).

In one embodiment of the present invention a process for the preparation of bisphenol compounds of formula (I), wherein the process comprising the steps of:
a. adding furfural to a phenolic compound in molar ratio of 1:2 in the presence of a basic catalyst followed by stirring of the reaction mixture at a temperature ranging between 25-35 oC for a period of time in the range of 1 to 6 h;
b. heating the reaction mixture obtained in step (a) at a temperature in the range of 70°C to 150°C for a period of time 1 to 6 to obtain bisphenol of formula (I).

In another embodiment of the present invention the phenolic compound is selected from syringol or guaiacol.

In another embodiment of the present invention the basic catalyst is either alkali metal hydroxide or alkali metal carbonate selected from NaOH, KOH or Na2CO3, K2CO3.

Still in another embodiment of the present invention the bisphenol compound of formula (I) is useful for the preparation of polyester wherein the process for the preparation of polyester comprises dissolving bisphenol compound of formula (I) in aqueous solution of sodium hydroxide followed by stirring at a temperature ranging between 0°C to 10°C for a period of time in the range of 1 to 4 h to get a reaction mixture, subsequently adding a catalyst and a solution of diacid chloride monomer in a solvent preferably dichloromethane with stirring speed ranging between 1500 to 2000 rpm for a period in the range of 1 to 6 h at a temperature ranging between 0°C to 10°C.

Still in another embodiment of the present invention the bisphenol compound of formula (I) is selected from the group consisting of 4, 4'-(furan-2-ylmethylene) bis(2,6-dimethoxyphenol) and 4,4'-(furan-2-ylmethylene)bis(2-methoxyphenol).

Still in another embodiment of the present invention catalyst is selected from the group consisting of benzyltriethylammonium chloride, tetraethylammonium chloride, tetramethylammonium chloride, benzyltriphenyl phosphonium chloride.

Still in another embodiment of the present invention diacid chloride monomer is selected from the group isophthaloyl chloride, terephthaloyl chloride, furan dicarboxylic acid chloride or mixture thereof.

Still in another embodiment of the present invention mole ratio of bisphenol compound of formula (I) and diacid chloride is 1:1

### BRIEF DESCRIPTION OF THE DRAWINGS:

**Figure 1****:** ¹H NMR spectrum of 4,4'-(furan-2-ylmethylene)bis(2,6-dimethoxyphenol) in CDCl₃.
**Figure 2****:** ¹³C NMR spectrum of 4,4'-(furan-2-ylmethylene)bis(2,6-dimethoxyphenol) in CDCl₃.

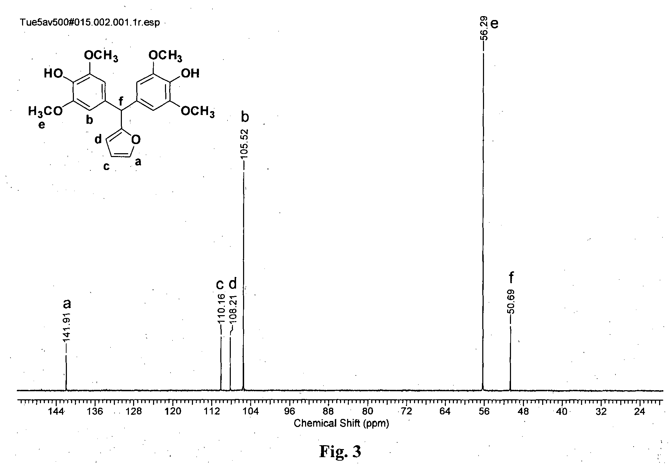

**Figure 3****:** DEPT spectrum of 4,4'-(furan-2-ylmethylene)bis(2,6-dimethoxyphenol) in CDCl₃.
**Figure 4****:** ¹H NMR spectrum of 4,4'-(furan-2-ylmethylene)bis(2-methoxyphenol) in CDCl₃
**Figure 5****:** ¹H NMR spectrum of polyester derived from 4, 4'-(furan-2-lmethylene)bis(2,6-dimethoxyphenol) and terephthaloyl chloride in CDCl₃
**Figure 6****:** ¹H NMR spectrum of polyester derived from 4, 4'-(furan-2-lmethylene)bis(2,6-dimethoxyphenol) and a mixture (50 : 50 mol %) of terephthaloyl chloride and isophthaloyl chloride in CDCl₃.

### DETAILED DESCRIPTION OF THE INVENTION:

The present invention provides novel bisphenols containing pendant furyl group of formula (I):

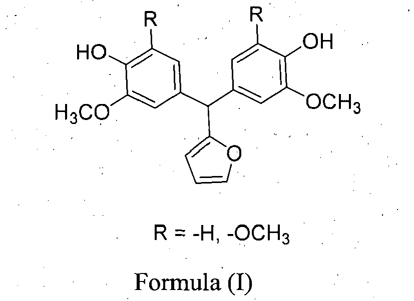

The compounds of formula (I) are selected from 4,4'-(furan-2-ylmethylene)bis(2-methoxyphenol) and 4, 4'-(furan-2-ylmethylene)bis(2,6-dimethoxyphenol).

Further, the present invention provides a process of synthesis of compounds of formula (I) by a catalyzed condensation of furfural with syringol or guaiacol.

In present invention, a process for the preparation of compounds of formula (I) comprising the steps of:
a) adding furfural to a mixture of phenolic compound and catalyst followed , by stirring at 30 °C for 4 h.
b) heating the reaction mixture of step (a) at 100°C for 4 h to afford desired bisphenol compounds of formula (I).

The process is catalyzed by base selected from alkali metal hydroxides or alkali metal carbonates selected from NaOH, KOH, Na₂CO₃, K₂CO₃, etc..

The process of synthesis of compounds of formula (I) by a catalyzed condensation of furfural with syringol or guaiacol is as depicts in scheme 1:

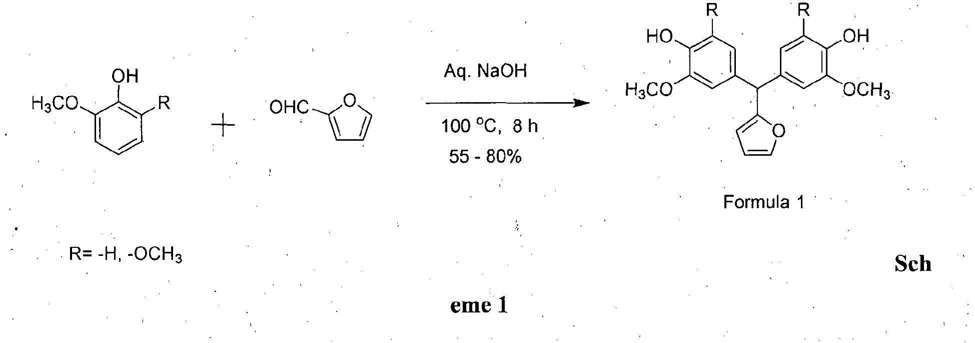

The present invention provides compounds of formula (I) which are used to prepare self-healable polymers selected from polycarbonates, polyesters, polyether sulfones, polyether ketones, epoxy resins, etc.

The compounds of formula (I) are useful difunctional monomers for the production of high performance polymers. Furthermore , 4,4'-(furan-2-ylmethylene)bis(2-methoxyphenol) and 4,4'-(furan-2-ylmethylene)bis(2,6-dimethoxyphenol)-based polymers contain pendant furyl groups which act as reactive sites for chemical modifications / crosslinking *via* click reaction with maleimides / bismaleimides.

The present invention provides a process for the preparation of polyester by polycondensation of compounds of formula (I) with a mixture (50: 50 mol %) of isophthaloyl chloride and terephthaloyl chloride.

The present invention provides a polymerization process for the preparation of polyesters based on compounds of formula (I) comprising the steps of:
a) dissolving compound of formula (I) in aqueous solution of sodium hydroxide;
b) stirring the reaction mixture of step (a) for 1 hr at 10°C followed by addition of benzyl triethyl ammonium chloride to the reaction mixture with constant stirring;
c) after 30 min, adding a solution of terephthaloyl chloride in dichloromethane to the reaction mixture of step (b) and the mixture was stirred vigorously at 2000 rpm for 1h followed by work up to afford desired polymer.

The following examples serve to provide the best modes of practice for the present invention, and should not be constructed as limiting the scope of the invention.

### EXAMPLES:

### Example 1: Synthesis of 4, 4'-(furan-2-ylmethylene)bis(2,6-dimethoxyphenol):

Into a 500 mL two necked round bottom flask equipped with a reflux condenser, an addition funnel and a magnetic stirring bar were charged syringol (10 g, 64.93 mmol) and 20% aqueous NaOH (2.5 mL, 5% by weight based on syringol) at 30 °C. Furfural (3.11 g, 32.40 mmol) was added dropwise with stirring. The reaction mixture was stirred at 30 °C for 4 h and then heated at 100°C for 4 h. The reaction mixture was cooled to at 30 °C and diluted with cold water (500 mL) with stirring. The solution was neutralized with dilute HCl. The precipitated product was separated out by filtration and washed with cold water. The product was dissolved in dichloromethane (200 mL) and the solution was dried over anhydrous sodium sulphate, filtered and dichloromethane was removed under reduced pressure at room temperature. The product was recrystallized from ethanol:water to afford pure 4,4'-(furan-2-ylmethylene)bis(2,6-dimethoxyphenol).

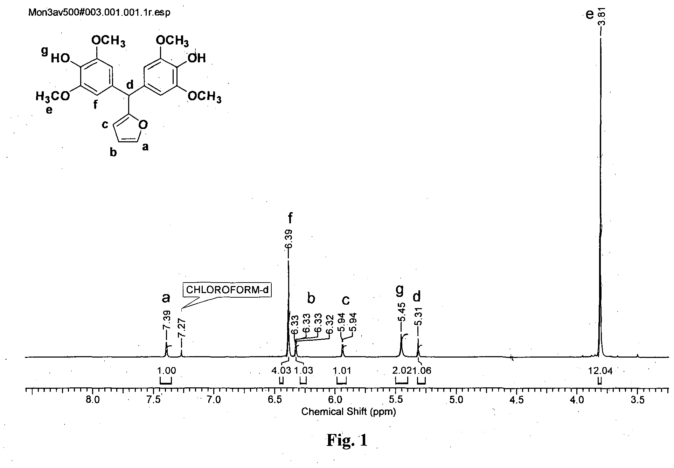

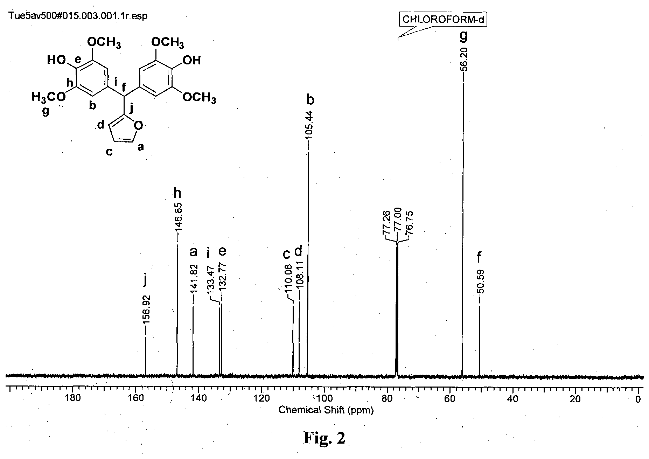

Yield 10.2 g (82%), M.P. 125°C. ¹H NMR (500 MHz, CDCl₃): Figure 1; δ ppm = 3.81 (s, 12 H) 5.31 (s, 1 H) 5.45 (br. s, 2 H) 5.94 (d, 1 H) 6.31-6.33 (m, 1 H) 6.39 (s, 4 H) 7.39 (br.s, 1 H). ¹³C NMR (500 MHz, CDCl₃): Figure 2; δ ppm = 50.69, 56.29, 105.53, 108.21, 110.16, 132.86, 133.56, 141.91, 146.95, 157.02 HRMS ESI⁺: [M⁺Na]⁺ m/z calculated for C₂₁H₂₂O₇ Na: 409.1264, found: 409.1258. Elemental analysis (%): calculated- C 65.58, H 5.74; found: C 65.05, H 5.84.

### Example 2: Synthesis of 4,4'-(furan-2-ylmethylene)bis(2-methoxyphenol):

Into a 500 mL two necked round bottom flask equipped with a reflux condenser, an addition funnel and a magnetic stirring bar were charged guaiacol (10 g, 80.61 mmol) and 20% aqueous NaOH (2.5 mL, 5% by weight based on guaiacol,) at 30 °C. Furfural (3.86 g, 40.30 mmol) was added dropwise with stirring. The reaction mixture was stirred at 30 °C for 4 h and then heated at 100°C for 4 h. The reaction mixture was cooled to 30 °C and diluted with cold water (500 mL) with stirring. The solution was neutralized with dilute HCl. The precipitated product was separated out by filtration and washed with cold water. The product was dissolved in dichloromethane (200 mL) and the solution was dried over anhydrous sodium sulphate, filtered and dichloromethane was removed under reduced pressure at room temperature. The product was recrystallized from ethanol:water to afford pure 4,4'-(furan-2-ylmethylene)bis(2-methoxyphenol).

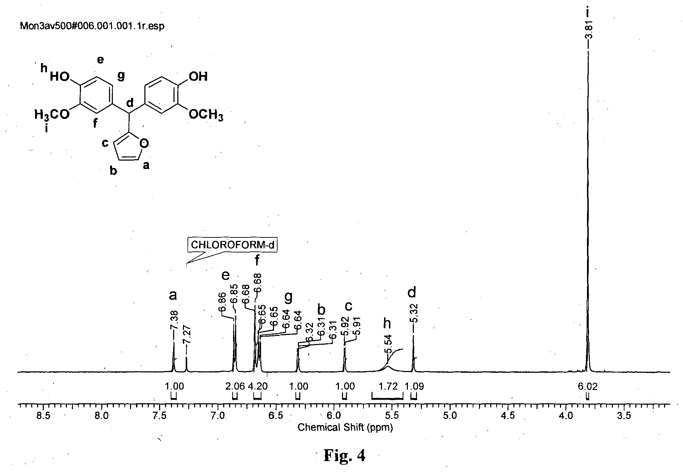

Yield 7.60 g (58%), M.P. 145°C. ¹H NMR (500 MHz, CDCl₃) Figure 4; δ ppm 3.81 (s, 6 H) 5.32 (s, 1 H) 5.54 (br. s, 2 H) 5.91(d, 1H) 6.30-6.32 (m 1 H) 6.64 (dd, 2 H) 6.68 (d, 2 H) 6.85 (d, 2 H) 7.38 (br.s, 1 H)

### Example 3: Synthesis of polyester by polycondensation of 4, 4'-(furan-2-ylmethylene)bis(2,6-dimethoxyphenol) with terephthaloyl chloride:

Polymerization reaction was carried out in a 100 mL two-necked round bottom flask equipped with a mechanical stirrer. 4, 4'-(furan-2-ylmethylene)bis(2,6-dimethoxyphenol) (1 g, 2.59 mmol) was dissolved in 5.5 mL of 1 M aqueous solution of sodium hydroxide. The mixture was stirred for 1h at 10°C. Next, benzyl triethyl ammonium chloride (30 mg) was added to the reaction mixture and stirring was continued. After 30 min, a solution of terephthaloyl chloride (0.525 g, 2.59 mmol) in 15 mL of dichloromethane was added to the reaction mixture and the mixture was stirred vigorously at 2000 rpm for 1h at 10°C. The reaction mixture was poured into hot water; the precipitated polymer was filtered and washed several times with water. The polymer was dissolved in chloroform and reprecipitated into methanol. The polymer was filtered, washed with methanol, and dried under reduced pressure at 50°C for 24 h.

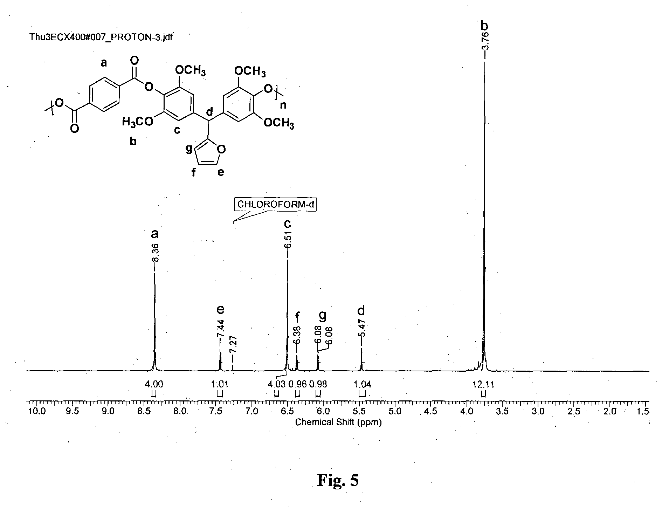

¹H NMR (200 MHz, CDCl₃) figure 5; δ ppm 3.76 (s, 12 H) 5.47 (s, 1 H) 6.08 (d, 1 H) 6.38 (br. s, 1 H) 6.51 (s, 4 H) 7.44 (d, 1 H) 8.36 (s, 4 H), Inherent viscosity (CHCl₃) - 0.29 dL/g, Molecular Weight: Mn - 14000 and Mw - 28000 (GPC in CHCl₃, Polystyrene standard), IR - 1744, 1213 cm⁻¹.

### Example 4: Synthesis of polyester by polycondensation of 4, 4'-(furan-2-ylmethylene)bis(2,6-dimethoxyphenol) with a mixture (50 : 50 mol %) of isophthaloyl chloride and terephthaloyl chloride:

Polymerization reaction was carried out in a 100 mL two-necked round bottom flask equipped with a mechanical stirrer. 4, 4'-(Furan-2-ylmethylene)bis(2,6-dimethoxyphenol) (1 g, 2.59 mmol) was dissolved in 5.5 mL of 1 M aqueous solution of sodium hydroxide. The mixture was stirred for 1h at 10°C. Next, benzyl triethyl ammonium chloride (30 mg) was added to the reaction mixture and stirring was continued. After 30 min, a solution of isophthaloyl chloride (0.262 g, 1.295 mmol) and terephthaloyl chloride (0.262 g, 1.295 mmol) in 15 mL of dichloromethane was added to the reaction mixture and the mixture was stirred vigorously at 2000 rpm for 1h at 10°C. The reaction mixture was poured into hot water; the precipitated polymer was filtered and washed several times with water. The polymer was dissolved in chloroform and reprecipitated into methanol. The polymer was filtered, washed with methanol, and dried under reduced pressure at 50°C for 24 h.

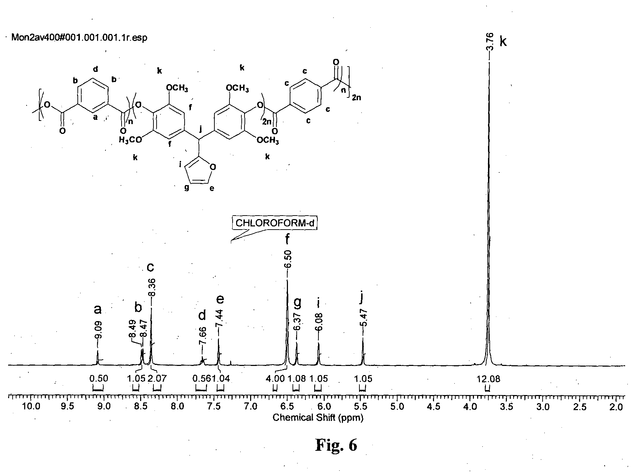

¹H NMR (400 MHz, CDCl₃) Figure 6; δ ppm 3.76 (s, Hₖ) 5.47 (s, Hⱼ) 6.08 (s, Hᵢ) 6.37 (s, H_{g}) 6.50 (s, H_{f}) 7.44 (s, Hₑ) 7.66 (t, H_{d}) 8.36 (s, H_{c}) 8.48 (d, H_{b}) 9.09 (s, Hₐ), Inherent viscosity (CHCl₃) - 1.50 dL/g, Molecular Weight- - Mn - 130000 and Mw- 230000 (GPC in CHCl₃, Polystyrene standard), IR - 1744, 1213 cm⁻¹.

### ADVANTAGES OF INVENTION:

- Raw materials are completely bio-based
- Bisphenols and polymers thereof contain pendant furyl group which provides reactive site for chemical modification.

## Claims

1. Bisphenol compounds of formula (I) 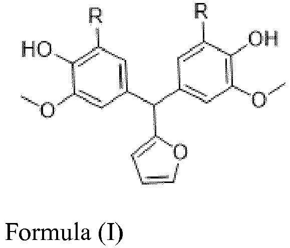 wherein R is hydrogen or methoxy.

2. The compound as claimed in claim 1, wherein the compounds are selected from 4, 4'-(furan-2-ylmethylene) bis(2,6-dimethoxyphenol) and 4,4'-(furan-2-ylmethylene)bis(2-methoxyphenol).

3. A process for the preparation of bisphenol compounds of formula (I) as claimed in claim 1, wherein the process comprising the steps of:
a. adding furfural to a phenolic compound in molar ratio of 1:2 in the presence of a basic catalyst followed by stirring of the reaction mixture at a temperature ranging between 25-35 °C for a period of time in the range of 1 to 6 h;
b. heating the reaction mixture obtained in step (a) at a temperature in the range of 70°C to 150°C for a period of time 1 to 6 to obtain bisphenol of formula (I).

4. The process as claimed in claim 3, wherein the phenolic compound is selected from syringol or guaiacol.

5. The process as claimed in claim 3, wherein the basic catalyst is either alkali metal hydroxide or alkali metal carbonate selected from NaOH, KOH or Na₂CO₃, K₂CO₃.

6. Use of the bisphenol compound of formula (I) as claimed in claim 1 for the preparation of polyester wherein the process for the preparation of polyester comprises dissolving bisphenol compound of formula (I) in aqueous solution of sodium hydroxide followed by stirring at a temperature ranging between 0°C to 10°C for a period of time in the range of 1 to 4 h to get a reaction mixture, subsequently adding a catalyst and a solution of diacid chloride monomer in a solvent preferably dichloromethane with stirring speed ranging between 1500 to 2000 rpm for a period in the range of 1 to 6 h at a temperature ranging between 0°C to 10°C.

7. The use as claimed in claim 6, wherein the bisphenol compound of formula (I) is selected from the group consisting of 4, 4'-(furan-2-ylmethylene) bis(2,6-dimethoxyphenol) and 4,4'-(furan-2-ylmethylene)bis(2-methoxyphenol).

8. The use as claimed in claim 6, wherein catalyst is selected from the group consisting of benzyltriethylammonium chloride, tetraethylammonium chloride, tetramethylammonium chloride, benzyltriphenyl phosphonium chloride.

9. The use as claimed in claim 6, wherein diacid chloride monomer is selected from the group isophthaloyl chloride, terephthaloyl chloride, furan dicarboxylic acid chloride or mixture thereof.

10. The use as claimed in claim 6, wherein mole ratio of bisphenol compound of formula (I) and diacid chloride is 1:1.

## Patentansprüche

1. Bisphenolverbindungen von Formel (I) 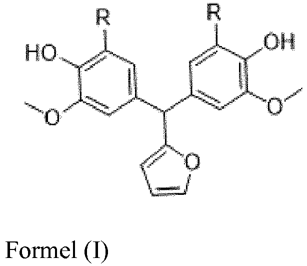 wobei R Wasserstoff oder Methoxy ist.

2. Verbindung nach Anspruch 1, wobei die Verbindungen aus 4,4'-(Furan-2-ylmethylen)bis(2,6-dimethoxyphenol) und 4,4'-(Furan-2-ylmethylen)bis(2-methoxyphenol) ausgewählt sind.

3. Verfahren zur Herstellung von Bisphenolverbindungen von Formel (I) nach Anspruch 1, wobei das Verfahren folgende Schritte umfasst:
a. Hinzufügen von Furfural zu einer Phenolverbindung in einem Molverhältnis von 1:2 in Gegenwart eines basischen Katalysators, gefolgt von Rühren des Reaktionsgemisches bei einer Temperatur im Bereich zwischen 25-35 °C für einen Zeitraum im Bereich von 1 bis 6 h;
b. Erhitzen des in Schritt (a) erhaltenen Reaktionsgemisches bei einer Temperatur im Bereich von 70 °C bis 150 °C für einen Zeitraum von 1 bis 6, um Bisphenol von Formel (I) zu erhalten.

4. Verfahren nach Anspruch 3, wobei die Phenolverbindung aus Syringol oder Guajacol ausgewählt ist.

5. Verfahren nach Anspruch 3, wobei der basische Katalysator entweder Alkalimetallhydroxid oder Alkalimetallcarbonat ist, das aus NaOH, KOH oder Na₂CO₃, K₂CO₃ ausgewählt ist.

6. Verwendung der Bisphenolverbindung von Formel (I) nach Anspruch 1 für die Herstellung von Polyester, wobei das Verfahren für die Herstellung von Polyester Lösen einer Bisphenolverbindung von Formel (I) in wässriger Lösung von Natriumhydroxid, gefolgt von Rühren bei einer Temperatur im Bereich zwischen 0 °C bis 10 °C für einen Zeitraum im Bereich von 1 bis 4 h, um ein Reaktionsgemisch zu erhalten, anschließend Hinzufügen eines Katalysators und einer Lösung von zweisäurigem Chloridmonomer in ein Lösungsmittel, vorzugsweise Dichlormethan, mit einer Rührgeschwindigkeit im Bereich zwischen 1500 bis 2000 rpm für einen Zeitraum im Bereich von 1 bis 6 h bei einer Temperatur im Bereich zwischen 0 °C bis 10 °C umfasst.

7. Verwendung nach Anspruch 6, wobei die Bisphenolverbindung von Formel (I) aus der Gruppe bestehend aus 4,4'-(Furan-2-ylmethylen)-bis(2,6-dimethoxyphenol) und 4,4'-(Furan-2-ylmethylen)bis(2-methoxyphenol) ausgewählt ist.

8. Verwendung nach Anspruch 6, wobei ein Katalysator aus der Gruppe bestehend aus Benzyltriethylammoniumchlorid, Tetraethylammoniumchlorid, Tetramethylammoniumchlorid, Benzyltriphenylphosphoniumchlorid ausgewählt ist.

9. Verwendung nach Anspruch 6, wobei zweisäuriges Chloridmonomer aus der Gruppe bestehend aus Isophthalsäuredichlorid, Terephthalsäuredichlorid, Furandicarbonsäurechlorid oder einer Mischung davon ausgewählt ist.

10. Verwendung nach Anspruch 6, wobei ein Molverhältnis der Bisphenolverbindung von Formel (I) zu zweisäurigem Chlorid 1:1 ist.

## Revendications

1. Composés bisphénol de formule (I) 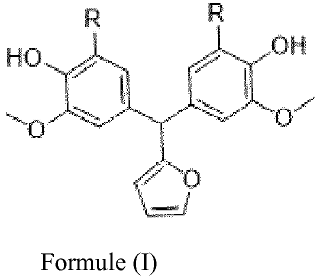 dans laquelle R est un atome d'hydrogène ou un groupe méthoxy.

2. Composé tel que revendiqué dans la revendication 1, les composés étant choisis parmi le 4,4'-(furan-2-ylméthylène) bis(2,6-diméthoxyphénol) et le 4,4'-(furan-2-ylméthylène)bis(2-méthoxyphénol).

3. Processus pour la préparation de composés bisphénol de formule (I) tels que revendiqués dans la revendication 1, le processus comprenant les étapes consistant à :
a. ajouter du furfural à un composé phénolique dans un rapport molaire de 1:2 en présence d'un catalyseur basique, puis agiter le mélange réactionnelle à une température dans la plage comprise entre 25 et 35 °C pendant une durée dans la plage de 1 à 6 h ;
b. chauffer le mélange réactionnel obtenu à l'étape (a) à une température dans la plage de 70 °C à 150 °C pendant une durée de 1 à 6 h pour obtenir du bisphénol de formule (I).

4. Processus tel que revendiqué dans la revendication 3, dans lequel le composé phénolique est choisi parmi le syringol ou le gaïacol.

5. Processus tel que revendiqué dans la revendication 3, dans lequel le catalyseur basique est soit un hydroxyde de métal alcalin, soit un carbonate de métal alcalin choisi parmi NaOH, KOH ou Na₂CO₃, K₂CO₃.

6. Utilisation du composé bisphénol de formule (I) tel que revendiqué dans la revendication 1 pour la préparation de polyester, dans laquelle le processus pour la préparation de polyester comprend la dissolution du composé bisphénol de formule (I) dans une solution aqueuse d'hydroxyde de sodium, suivie par une agitation à une température dans la plage comprise entre 0 °C à 10 °C pendant une durée dans plage de 1 à 4 h pour obtenir un mélange réactionnel, puis l'ajout d'un catalyseur et d'une solution de monomère de chlorure de diacide dans un solvant, de préférence le dichlorométhane, avec une vitesse d'agitation comprise entre 1500 à 2000 tr/min pendant une durée dans la plage de 1 à 6 h à une température dans la plage comprise entre 0 °C à 10 °C.

7. Utilisation telle que revendiquée dans la revendication 6, dans laquelle le composé bisphénol de formule (I) est choisi dans le groupe constitué par le 4,4'-(furan-2-ylméthylène) bis(2,6-diméthoxyphénol) et le 4,4'-(furan-2-ylméthylène)bis(2-méthoxyphénol).

8. Utilisation telle que revendiquée dans la revendication 6, dans laquelle le catalyseur est choisi dans le groupe constitué par le chlorure de benzyltriéthylammonium, le chlorure de tétraéthylammonium, le chlorure de tétraméthylammonium, le chlorure de benzyltriphényl phosphonium.

9. Utilisation telle que revendiquée dans la revendication 6, dans laquelle le monomère de chlorure de diacide est choisi dans le groupe du chlorure d'isophtaloyle, du chlorure de téréphtaloyle, du chlorure d'acide furan-dicarboxylique ou de mélanges de ceux-ci.

10. Utilisation telle que revendiquée dans la revendication 6, dans laquelle le rapport molaire du composé bisphénol de formule (I) et du chlorure de diacide est de 1:1.
